(19) Europäisches Patentamt
European Patent Office
Office européen des brevets

(11) Veröffentlichungsnummer: **0 013 376**
**B1**

(12) # EUROPÄISCHE PATENTSCHRIFT

(45) Veröffentlichungstag der Patentschrift: **19.05.82**

(21) Anmeldenummer: **79105142.8**

(22) Anmeldetag: **13.12.79**

(51) Int. Cl.³: **C 07 D 261/18,**
A 61 K 31/42,
C 07 C 103/375,
C 07 C 103/58

(54) **Ein Isoxazolderivat, Verfahren zu seiner Herstellung und diese Verbindung enthaltende Mittel.**

(30) Priorität: **16.12.78 DE 2854439**

(43) Veröffentlichungstag der Anmeldung:
**23.07.80 Patentblatt 80/15**

(45) Bekanntmachung des Hinweises auf die Patenterteilung:
**19.05.82 Patentblatt 82/20**

(84) Benannte Vertragsstaaten:
**AT BE CH DE FR GB IT LU NL SE**

(56) Entgegenhaltungen:
**DE - A - 2 524 959**
**DE - A - 2 654 797**

(73) Patentinhaber: **HOECHST AKTIENGESELLSCHAFT**
**Postfach 80 03 20**
**D-6230 Frankfurt/Main 80 (DE)**

(72) Erfinder: **Kämmerer, Friedrich-Johannes, Dr.**
**Am Weiher 27**
**D-6203 Hochheim am Main (DE)**
Erfinder: **Schleyerbach, Rudolf, Dr.**
**Finkenweg 10**
**D-6238 Hofheim am Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 013 376 B1

Ein Isoxazolderivat, Verfahren zu seiner Herstellungünd diese Vervindung enthaltende Mittel

Nach DE-OS 25 24 959 sind 5-Methylisoxazol-4-carbonsäureanilide mit antiphlogistischen und analgetischen Wirkungen bekannt. Bei der Untersuchung ähnlicher Verbindungen zeigte sich nun, daß man bei Einführung einer Trifluormethylgruppe in die 4-Stellung des Anilidteils eine Verbindung erhält, die sowohl hinsichtlich ihrer Wirkstärke und therapeutischen Breite als auch ihres Wirkungsprofils den bekannten 5-Methyl-isoxazol-4-carbonsäureaniliden deutlich überlegen ist.

Gegenstand der Erfindung ist 5-Methylisoxazol-4-carbonsäure-(4-trifluormethyl)-anilid der Formel I

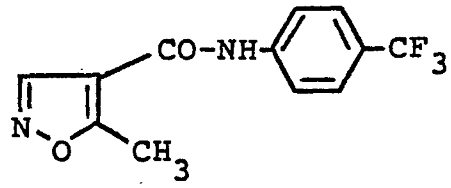

(I)

Gegenstand der Erfindung ist auch ein Verfahren zur Herstellung der Verbindung der Formel I, das dadurch gekennzeichnet ist, daß man 4-Trifluormethylanilin der Formel II

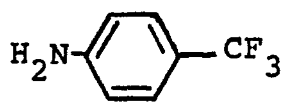

(II)

a) mit einem 5-Methylisoxazol-4-carbonsäure-Derivat der Formel III

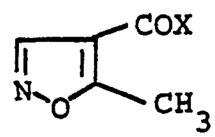

(III)

in der X entweder ein Halogenatom, vorzugsweise Chlor oder Brom, oder eine YO— oder ZO—CO—O—Gruppe bedeutet, wobei Y für gegebenenfalls durch Fluor, Chlor, Brom, Jod, Methyl, Äthyl, Methoxy, Äthoxy, Trifluormethyl, Nitro oder Cyan einfach, zweifach oder dreifach subsituiertes Phenyl oder für den Acylrest entsprechend Formel III (Formel III ohne X) und Z für ($C_1$—$C_4$)-Alkyl, Phenyl oder Benzyl stehen, umsetzt, oder

b) mit Diketen oder einem reaktiven Acetessigsäurederivat umsetzt, das so erhaltene Acetessigsäureanilid der Formel IV

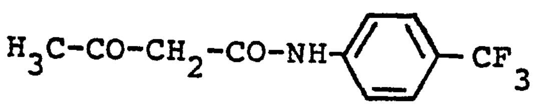

(IV)

mit einem Orthoameisensäureester der Formel V

$$HC(OR)_3 \qquad (V)$$

worin R ($C_1$—$C_4$)-Alkyl bedeutet, und mit einem Säureanhydrid erwärmt und das so erhaltene 2-Alkoxymethylenacetessigsäureanilid der Formel VI

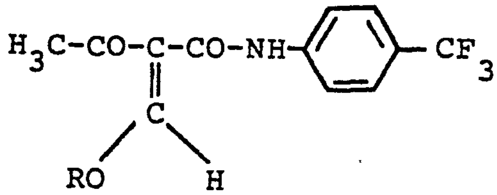

(VI)

worin R die oben genannten Bedeutungen hat, mit Hydroxylamin umsetzt.

Die Reaktion nach Verfahrensvariante a) wird zweckmäßig in einem Verteilungs- oder Lösungsmittel durchgeführt, das sich unter den Reaktionsbedingungen gegenüber den Reaktionspartnern indifferent verhält. Hierfür kommen beispielsweise Nitrile, wie Acetonitril, Äther, wie Diäthyläther, Tetrahydrofuran oder Dioxan und Alkohole, wie Methanol, Athanol, Propanol oder Isopropanol und Wasser in Frage.

In einer bevorzugten Ausführungsform der Verfahrensvariante a) wird Trifluormethylanilin der Formel II mit dem Carbonsäurechlorid der Formel III, zweckmäßig in Gegenwart eines säurebindenden Mittels, wie Kalium- oder Natriumcarbonat, Alkali- oder Erdalkalihydroxid oder -alkoholat, einer organischen Base, wie Triäthylamin, Pyridin, Picolin oder Chinolin oder des im Überschuß eingesetzten Anilins der Formel II bei Temperaturen zwischen 0 und 160°C, vorzugsweise zwischen 20 und 80°C, umgesetzt. Die Reaktionszeiten können von wenigen Minuten bis zu zwei Stunden betragen.

Die als Ausgangsstoffe benötigten-5-Methylisoxazol-4-carbonsäure-Derivate der Formel III werden entsprechend DRP 634 286 durch Umsetzung von Äthoxymethylenacetessigester mit Hydroxylamin zum 5-Methylisoxazol-4-carbonsäureester, saures Verseifen des so erhaltenen Esters, z.B. in einem Gemisch aus Eisessig und konzentrierter Salzsäure im Verhältnis 2 : 1, zur 5-Methylisoxazol-4-carbonsäure und Überführen dieser Carbonsäure nach üblichen Methoden in die Carbonsäurehalogenide, Ester oder Anhydride, erhalten.

Zur Durchführung der Verfahrensvariante b) wird das 4-Trifluormethylanilin der Formel II mit einer zweckmäßig mindestens äquimolaren Menge Diketen oder eines reaktiven Acetessigsäurederivates, z.B. eines ($C_1$—$C_4$)-Alkyl- oder Acrylacetoacetats, vorzugsweise Methyl-, Äthyl-, Phenyl- oder 2,4-Dichlorphenylacetoacetats, oder eines Acetessigsäurehalogenids, zweckmäßig in einem Lösungs- oder Verteilungsmittel, das sich unter den Reaktionsbedingungen gegenüber den Reaktionspartnern indifferent verhält, z.B. einem Nitril wie Acetonitril oder einem Ather, wie Diäthyläther, Tetrahydrofuran oder Dioxan, bei Temperaturen zwischen 20° und 100°C, vorzugsweise zwischen 40° und 80°C, und während Reaktionszeiten von 10 Minuten bis zu 3 Stunden umgesetzt, das so erhaltene Acetessigsäureanilid der Formel IV mit einer zweckmäßig mindestens äquimolaren Menge eines Orthomeisensäureesters der Formel V und vorteilhaft in einem 2- bis 4-fach molaren Überschuß an einem Säureanhydrid, zweckmäßig einem aliphatischen Säureanhydrid mit 4 bis 6 C-Atomen, vorzugsweise Acetanhydrid, 30 Minuten bis 3 Stunden lang auf eine Temperatur zwischen 80° und 150°C, vorzugsweise auf die Siedetemperatur des Gemisches erwärmt, das so erhaltene 2-Alkoxymethylenacetessigsäureanilid der Formel VI isoliert und anschließend mit einer zweckmäßig mindestens äquimolaren Menge Hydroxylamin in einem organischen Lösungsmittel oder Lösungsmittelgemisch, vorzugsweise Methyl-, Äthyl-, Propyl- oder Isopropylalkohol, gegebenenfalls unter Zusatz von bis zu 2 Vol.-Teilen, vorzugsweise bis zu 1 Vol.-Teil, Wasser auf 1 Vol.-Teil organisches Lösungsmittel, bei einer Temperatur zwischen 0° und 130°C, vorzugsweise zwischen 20° und 100°C umgesetzt. Die Reaktionszeiten liegen zwischen wenigen Minuten und 5 Stunden.

Das zur Durchführung der Verfahrensvariante b) als Zwischenprodukt verwendete Acetessigsäure-(4-trifluormethyl)-anilid der Formel IV und die 3-Alkoxymethylenacetessigsäureanilide der Formel VI sind neu. Sie wirken analgetisch und antipyretisch und sind daher als Arzneimittel verwendbar.

Das Verfahrensprodukt der Formel I fällt aus der Reaktionsmischung — gegebenenfalls nach Abfiltrieren der Nebenprodukte und Einengen des Filtrats-kristallin aus, sofern mit organischen Lösungsmitteln gearbeitet wird. Aus einer wäßrigen Reaktionsmischung wird das Produkt durch Extrakion mit einem polaren organischen Lösungsmittel wie Methylenchlorid, Chloroform oder Trichloräthylen und Einengen bzw. Eindampfen des Extraktes erhalten. Das Produkt kann anschließend durch Umkristallisieren gereinigt werden. Dazu verwendet man ein organisches, vorzugsweise mäßig polares Lösungsmittel wie Toluol, ein Dimethylbenzol, Benzol, Cyclohexan, Methanol oder Äthanol, oder ein Gemisch aus solchen Lösungsmitteln.

Die erfindungsgemäße Isoxazolverbindung der Formel I kann auf Grund ihrer pharmakologischen Eigenschaften als Arzneimittel, insbesondere als Antirheumatikum, Antiphlogistikum, Antipyretikum und Analgeticum sowie zur Behandlung der multiplen Skelrose Verwendung finden. Sie kann entweder allein, gegebenenfalls in Form von Mikrokapseln, oder vermischt mit geeigneten Trägerstoffen verabreicht werden. Gegenstand der Erfindung sind somit auch Arzneimittel, die aus der Verbindung der Formel I bestehen oder diesen Wirkstoff neben einen üblichen physiologisch verträglichen Trägerstoff, Verdünnungsmittel und/oder Konstituens enthalten. Die Mittel können oral, rectal oder parenteral appliziert werden, wobei die orale oder rectale Anwendung bevorzugt ist. Geeignete feste oder flüssige galenische Zubereitungsformen sind beispielsweise Granulate, Pulver, Tabletten, Kapseln, Suppositorien, Sirupe, Suspensionen oder Tropfen sowie Präparate mit protrahierter Wirkstofffreigabe. Als häufig verwendete Trägermittel seien z.B. Calciumcarbonat, Calciumphosphate, verschiedene Zucker oder Stärkearten, Cellulosederivative, Gelatine, pflanzliche Öle, Polyäthylenglykole und physiologisch unbedenkliche Lösungsmittel genannt.

Eine weitere Anwendung der Verbindung der Formel I besteht in der Kombination mit anderen geeigneten Wirkstoffen, beispielsweise Antiuricopathika, Trombocytenaggregationshemmern, anderen Analgetika und anderen steroidalen oder nichtsteroidalen Antiphlogistika.

*Herstellungsbeispiele* für das 5-Methylisoxazol-4-carbonsäure-(4-trifluormethyl)-anilid der Formel I

Verfahrensvariante a):

1. 0,1 Mol 4-Trifluormethylanilin (II) (16,1 g), gelöst in 150 ml Acetonitril, werden bei Raumtemperatur tropfenweise mit einer Lösung von 0,05 Mol 5-Methyl-isoxazol-4-carbonsäurechlorid (III) (7,3 g) in 20 ml Acetonitril unter Rühren versetzt. Nach weiteren 20 Minuten Rühren wird das ausgefallene 4-Trifluormethyl-anilin-Hydrochlorid abgesaugt, zweimal mit je 20 ml Acetonitril gewaschen und die vereinigten Filtrate unter vermindertem Druck eingeengt. Man erhält so 12,8 g (94.8 $ d.Th.) weißes, kristallines 5-Methyl-isoxazol-4-carbonsäure-(4-trifluormethyl)-anilid der Formel I, Schmelzpunkt (aus Toluol) 166,5°C.

2. 0,1 Mol Trifluormethylanilin (II) (16,1 g) suspendiert in 150 ml Wasser, werden tropfenweise mit 0,1 Mol 5-Methylisoxazol-4-carbonsäurechlorid (III) (14,6 g) und 20 ml einer 5 n Kalilauge so versetzt, daß der pH-Wert der Reaktionsmischung nicht über 5 steigt. Diese wird anschlißend mit 150 ml Methylenchlorid geschüttelt. Die Methylenchloridphase wird mit Wasser gewaschen und nach dem Trocknen mit Natriumsulfat unter vermindertem Druck zur Trockne gebracht. Man erhält so 24,4 g (90,2% d.Th.) kristallines Produkt der Formel I, Schmelzpunkt (aus Toluol) 166,5°C.

3. In eine Lösung von je 0,1 Mol 4-Trifluormethylanilin (II) (16,1 g) und Triäthylamin (5,06) in 300 ml Acetonitril läßt man 0,1 Mol 5-Methylisoxazol-4-carbonsäurechlorid (III) (14,6 g) gelöst in 20 ml Acetonitril unter Rühren zutropfen. Es wird 20 Minuten nachgerührt. Die augefallenen Nebenprodukte werden abgesaugt. Das Filtrat wird unter vermindertem Druck zur Trockne gebracht. Der Rückstand wird mit je 300 ml Methylenchlorid und Wasser ausgechüttelt. Die Methylenchloridphase wird mit Wasser gewaschen, getrocknet und unter vermindertem Druck eingeengt. Man erhält so 21,2 g (78,1% d.Th.) kristallines Produkt der Formel I, Schmelzpunkt (aus Toluol) 166,5°C.

4. 0,1 Mol (4-Trifluormethylanilin (II) (16,1 g) werden mit 0,1 Mol Äthoxycarbonyl-5-methylisoxazol-4-carboxylat (III) (19,9 g) in 80 ml Tetrahydrofuran 1 Stunde unter Rückfluß gekocht. Man engt die Reaktionsmischung unter vermindertem Druck ein. Beim Erkalten fällt das Produkt kristallin aus. Weitere Mengen erhält man aus dem Rückstand durch Kristallisieren aus Toluol: 5-Methylisoxazol-4-carbonsäure-(4-trifluormethyl)-anilid vom Schmelzpunkt 166,5°C.

5. 0,1 Mol 4-Trifluormethylanilin (II) (16,1 g) und 0,1 Mol Benzyloxycarbonyl-5-methylisoxazol-4-carboxylat (III) (26,1 g) werden in 150 ml Dioxan gelöst und 90 Minuten unter Rückfluß erhitzt. Man dampft das Lösungsmittel unter vermindertem Druck ab. Aus dem Rückstand erhält man nach Umkristallisieren aus Toluol 5-Methylisoxazol-4-carbonsäure-(4-trifluormethyl)-anilid vom Schmelzpunkt 166,5°C.

6. 0,1 Mol 4-Trifluormethylanilin (II) (16,1 g) und 0,1 Mol (2,4-Dichlor)phenyl-5-methylisoxazol-4-carboxylat (III) (27,2 g), gelöst in 150 ml Tetrahydrofuran, werden 2 Stunden unter Rückfluß erhitzt. Man dampft die Mischung unter vermindertem Druck zur Trockne ein und gewinnt aus dem Rückstand nach Umkristallisieren aus Toluol 5-Methylisoxazol-4-carbonsäure-(4-trifluormethyl)-anilid vom Schmelzpunkt 166,5°C.

7. 0,1 Mol 4-Trifluormethylanilin (II) (16,1 g) werden mit 0,1 Mol 5-Methylisoxazol-4-carbonsäureanhydrid (III) (23,6 g) in 150 ml Acetonitril 2 Stunden unter Rückfluß erhitzt. Man engt die Mischung unter vermindertem Druck stark ein und erhält nach Kristallisieren aus Toluol 5-Methylisoxazol-4-carbonsäure-(4-trifluormethyl)-anilid vom Schmelzpunkt 166,5°C.

Verfahrensvariante b):

*Stufe 1:*

Acetessigsäure-4-trifluormethylanilid der Formel IV

In eine Lösung von 0,5 Mol 4-Trifluormethylanilin (II) (30,6 g) in 150 ml Acetonitril läßt man bei 75°C eine Mischung von 0,55 Mol Diketen (46,3 g) in 30 ml Acetonitril zutropfen. Es wird 2,5 Stunden unter Rückfluß zum Sieden erhitzt. Nach dem Abkühlen auf Zimmertemperatur werden die ausgefallenen Kristalle abgesaugt, mit kaltem Äthanol gewaschen und getrocknet. Man erhält so 79,1 g (64,5% d.Th.) kristallines Acetessigsäure-4-trifluormethylanilid der Formel IV, Schmelzpunkt (aus Äthanol) 155°C.

Die Acetonitrilphase wird unter vermindertem Druck zur Trockne gebracht. Der kristalline Rückstand (42,1 g) wird aus 80 ml Äthanol umkristallisiert. Man erhält so weitere 24,1 g (19,7% d.Th.) Kristalle. Schmelzpunkt (aus Äthanol) 155°C. Gesamtausbeute: 84,2% d.Th.

*Stufe 2:*

2-Äthoxymethylenacetessigsäure-4-trifluormethylanilid der Formel VI

0,75 Mol Acetessigsäure-4-trifluormethylanilid (183,9 g) aus Stufe 1 werden mit 0,83 Mol Orthoameisensäuretriäthylester (V) (123 g) und 2,25 ml Acetanhydrid (229,7 g) 1,5 Stunden unter Rückfluß gekocht. Nach dem Abkühlen auf Zimmertemperatur werden die ausgefallenen Kristalle abgesaugt und zunächst mit wenig Acetanhydrid und dann mit Petroläther gewaschen. Man erhält so 116,1 g (51,4% d.Th.) kristallines 2-Äthoxymethylenacetessigsäure-4-trifluormethylanilid der Formel VI, Schmelzpunkt (aus Toluol) 124—125°C.

Die vereinigten Filtrate werden unter vermindertem Druck eingeengt. Die Kristalle des dabei zurückbleibenden Kristallbreies werden abgesaugt, zunächst mit wenig Acetanhydrid und dann mit Petroläther gewaschen und getrocknet. Man erhält weitere 56,1 g (24,8% d.Th.) Kristalle. Schmelzpunkt (aus Toluol) 124 bis 125°C. Gesamtausbeute: 76,2% d.Th.

*Stufe 3:*

5-Methylisoxazol-4-carbonsäure-(4-trifluormethyl)-anilid der Formel I

0,11 Mol Hydroxylaminhydrochlorid (7,65 g) werden in 50 ml Wasser gelöst und mit einer eiskalten Lösung von 0,11 Mol Natriumhydroxid (4,4 g) in 10 ml Wasser versetzt. Dann läßt man in diese Hydroxylaminlösung bei 5 bis 10°C 0,1 Mol 2-Äthoxymethylenacetessigsäure-4-trifluormethylanilid der Formel VI (30,1 g) aus Stufe 2, gelöst in 60 ml Äthanol, zutropfen. Man erhitzt anschließend 15 Minuten unter Rückfluß. Die nach dem Abkühlen ausgefallenen Kristalle werden abgesaugt, mit Wasser gewaschen und getrocknet. Man erhält so 19,6 g (72,6% d.Th.) kristallines 5-Methylisoxazol-4-carbonsäure-(4-trifluormethyl)-anilid der Formel I), Schmelzpunkt (aus Toluol) 166,

Pharmakologische Prüfung und Ergebnisse

Das 5-Methylisoxazol-4-carbonsäure-(4-trifluormethyl)-anilid der Formel I gemäß der Erfindung [Verbindung A] wurde mit den nach DE - OS 25 24 959, Tabelle 2, Nr. 10, 11 und 12 bekannten chemisch nahestehenden Isoxazolderivaten 5-Methylisoxazol-4-carbonsäure-(4-fluor)-anilid [Verbindung B], 5-Methylisoxazol-4-carbonsäure-(3-trifluormethyl)-anilid [Verbindung C], 5-Methylisoxazol-4-carbonsäure-(3,5-bis-trifluormethyl)-anilid [Verbindung D], sowie mit dem 5-Methylisoxazol-4-carbonsäure-(2-trifluormethyl)-anilid [Verbindung E] und mit dem bekannten Antiphlogistikum Phenylbutazon [Verbindung F] in den anschließend beschriebenen Tiermodellen vergleichend auf antiphlogistische Wirkung, den Einfluß auf immunpathologische Prozesse, die ulcerogene Aktivität und akute Toxizität geprüft. Die Ergebnisse dieser Untersuchungen sind in den nachstehenden Tabellen 1 und 2 zusammengefaßt. Danach ist die Verbindung gemäß der Erfindung den bekannten Verbindungen in überraschendem Maß überlegen.

*1. Adjuvans-Arthritis, Praeventiv-Versuch*

Die Untersuchungen wurden nach der Methode von Pearson durchgeführt (Arthrit. Rheum. *2*, 440 (1959)). Als Versuchstiere dienten männliche Ratten eines Wistar-Lewis-Stammes im Körpergewicht zwischen 130 und 200 g. Die zu vergleichenden Verbindungen wurden täglich vom 1. bis zum 17. Versuchstag oral appliziert. Tiere einer Kontrollgruppe erhielten nur das Lösungsmittel. Pro Dosierung und in der Kontrollgruppe wurden jeweils 8 Tiere verwendet. Als Wirkungskriterium diente die Herabsetzung der Pfotenvolumenzunahme gegenüber der unbehandelten Kontrollgruppe. Die $ED_{50}$-Werte wurden graphisch aus der Dosiswirkungskurve bestimmt.

*2. Adjuvans-Arthritis, Modifikation nach Perper*

(Proc. Soc. exp. Biol. Med. *137*, 506 (1971)

Versuchstiere und Versuchsanordnung wie unter 1., jedoch wurden die Tiere lediglich vom 1. bis zum 12. Versuchstag behandelt; nach einem behandlungsfreien Intervall von 9 Tagen erfolgte die Bestimmung des Pfotenvolumens am 21. Tag. In diesem Test sind bekannte nichtsteroidale Antiphlogistika unwirksam, da sie lediglich symptomatische während der Zeit ihrer Gabe wirken und die bei der Adjuvans-Arthritis zugrunde liegenden immunpathologischen Prozesse nicht beeinflussen, so daß das Krankheitsbild sich bis zum 21. Tag voll entfaltet.

*3. Allergische Encephalomyelitis*

Dieses immunpathologische Krankheitsbild zeigt wie die Adjuvans-Arthritis eine Reihe von Parallelen mit bestimmten Erkrankungen des rheumatischen Formenkreises (vgl. P.A. Miescher and H.-J. Müller-Eberhard, Text-book of Immunopathology, Grune and Stratton, New York p. 179 et seq. (1976)). Bekannte nicht-steroidale Antiphlogistika sind hier allenfalls sehr schwach wirksam.

Die Untersuchungen wurden in der Methodik nach Levine durchgeführt (Arch. int. Pharmacodyn. *230*, 309 (1977)). Als Versuchstiere dienten männliche Ratten eines Wistar-Lewis-Stammes im Körpergewicht zwischen 180 bis 220 g. Die Encephalomyelitis wurde induziert durch Injektion von komplettem Freund' schem Adjuvans, dem frisch entnommener homogenisierter Rattenrückenmark-Extrakt sowie Bordetella-Pertussis-Vaccine zugesetzt waren. Die zu vergleichenden Verbindungen wurden täglich einmal oral vom 1. bis zum 12. Tag appliziert. Pro Dosierung und in der Kontrollgruppe wurden jeweils 10 Tiere verwendet. Ab dem 7. Versuchstag wurden die Lähmungssymptome unter Einbeziehung der Mortalität und der Körpergewichtsentwicklung täglich in einem Index zusammengefaßt.

Hierfür gilt:

| | |
|---|---|
| Verlust von je 20 g Körpergewicht | — 1 Punkt |
| Schwanzlähmung | — 1 Punkt |
| Nachhandlähmung | — 3 Punkte |

| | |
|---|---|
| Lähmung des ganzen Körpers | — 5 Punkte |
| Tod | — 5 Punkte |

Als Beurteilungskriterium diente die prozentuale Hemmung des Index gegenüber unbehandelten Kontrolltieren am 11. Versuchstag. Bei Abschluß des Versuches wurde den überlebenden Tieren Blut zur Bestimmung von Erythrozyten- und Leukoxytenzahlen sowie des Haematokrits entnommen.

*4. Akute ulzerogene Wirkung*

Die Untersuchungen erfolgten an jeweils 10 männlichen Sprague-Dawley Ratten im Körpergewicht von 200—300 g. 48 Stunden vor Applikation der zu vergleichenden Verbindungen bis zum Töten der Tiere wurde das Futter entzogen, bei freiem Zugang zum Trinkwasser. Die Ratten wurden 24 Stunden nach oraler Gabe getötet, der Magen entnommen, unter fließendem Wasser gereinigt und auf Schleimhautläsionen inspiziert. Als Ulzera galten alle makroskopisch sichtbaren Läsionen. Bestimmt wurde der Anteil der Tiere mit Ulzera pro dosi und die $UD_{50}$ nach Litchfield und Wilcoxon (J. Pharmacol. exp. Ther. *96*, 99 (1949)).

*5. Subakute ulzerogene Wirkung*

Versuchstiere und Versuchsdurchführung wie unter 4. mit folgender Abweichung: Die zu vergleichenden Verbindungen wurden täglich einmal oral über 4 Tage an normal gefütterte Ratten appliziert und die Tiere am 5. Tag nach 24 Stunden Futterentzung getötet. Diese Methodik verfügt nach Shriver über eine höhere Aussagekraft als die einmalige Applikation, da auch die medikamentöse Therapie von Rheumapatienten eine mehrmalige Behandlung erfordert (Toxicology and Appl. Pharmacology *32*, 73 (1975)).

*6. Akute Toxizität*

Die Bestimmung der akuten Toxizitäten erfolgte in Standardmethoden an NMRI-Mäusen, sowie an Ratten der Wistar- und Sprague-Dawley-Stämme. Pro Dosierung wurden 6 Tiere verwendet. Die $LD_{50}$-Werte wurden nach Litchfield und Wilcoxon bestimmt.

TABELLE 1

| Verbindung | $ED_{50}$ (mg/kg) Adjuvans-Arthritis Preavent. (1) | Perper (2) | Allerg. Encep. (3) Dosis mg/kg | % Hemmung | Ulzerogenität (4) akut $UD_{50}$ (mg/kg) | (5) subakut $UD_{50}$ (mg/kg) | Therap. Index akut $\frac{UD\ 50}{ED\ 50}$ | subakut $\frac{UD\ 50}{ED\ 50}$ |
|---|---|---|---|---|---|---|---|---|
| A | 2,3 | 4,5 | 10 | 100 | 57 (40–82) | 77 (52–113) | 25 | 33 |
| B | 15,0 | 25,0 | 25<br>50 | 43 Anämie | 250 (231–270) | 118 (97–144) | 17 | 8 |
| C | >50,0 | bis 20,0 unwirksam | 10 | 0 | im Bereich d. $LD_{50}$ ∾600 | | <12 | |
| D | ∾50,0 | >50,0 | | | 100 | | ∾2 | |
| E | >50,0 | bis 20,0 unwirksam | 10 | 0 | 350 (177–693) | | <7 | |
| F | 37,0 | bis 75 unwirksam | 75 | | 100 | | 3 | |

TABELLE 2

| Verbindung | Akute Tox. i.p. Maus | Akute Toxizität per os $LD_{50}$ (mg/kg) | Spezies, Stamm | Therap. Index $\frac{LD50}{ED50}$ |
|---|---|---|---|---|
| A | 150–300 | 235 (167–332) | Ratte | 100 |
| B | 200–400 | 620 (529–727) | Ratte Lewis | 41 |
| C | 100–250 | 740 (624–878) | Maus NMRI | <15 |
| D | >500 | 2530 (2162–2960) | Maus NMRI | ~ 50 |
| E | 300–600 | | | |
| % | | 780 | Ratte | 21 |

Die erfindungsgemäße Verbindung der Formel I erweist sich somit in den folgenden pharmakologischen Eigenschaften den Verbindungen nach DE - OS 25 24 959 überlegen:

1. Sie weist eine 6,5 mal höhere Wirkungsstärke ($ED_{50}$, Adjuvans-Arthritis, praeventiv) als 5-Methyl-isoxazol-4-carbonsäure-(4-fluor)-anilid (Verbindung B) auf.

2. Sie zeigt gegenüber bekannten Verbindungen aus DE - OS 25 24 959 eine überlegene therapeutische Breite:
a) hinsichtlich der Nebenwirkungen auf die gastrale Mukosa, insbesondere nach mehrmaliger Gabe: 4,1 mal höherer therapeutischer Index ($UD_{50}/ED_{50}$; subakut) gegenüber der Verbindung B;
b) hinsichtlich der oralen Toxizität: die therapeutische Breite ($LD_{50}/ED_{50}$) ist 2,4 mal höher als die der Verbindung B.

3. Sie inhibiert immunpathologische Prozesse in der Adjuvans-Arthritis nach Perper und in der allergischen Encephalomyelitis im therapeutischen Dosisbereich. Dies gelingt mit den Vergleichsverbindungen nur in viel geringerem Maße; insbesondere gelingt mit ihnen keine 100%ige Inhibition der Lähmungssymptomatik bei der allergischen Ecephalomyelitis ohne gastro-intestinalen Blutverlust, wie es mit dem erfindungsgemäßen Präparat möglich ist.

Die vorstehenden pharmakologischen Befunde zeigen, daß die erfindungsgemäße Verbindung der Formel I sich im Wirkungsprofil von den geprüften Antiphlogistika vorteilhaft unterscheidet, und zwar insbesondere durch die Hemmung immunpathologischer Prozesse an Tiermodellen, die auch für die menschliche Erkrankung Relevanz haben. Das dürfte in gleicher Weise gegenüber anderen bisher in der Therapie eingesetzten Antiphlogisitika gelten. Diese Tatsache eröffent die Möglichkeit, mehr ursächlich die rheumatischen Erkrankungen des Menschen medikamentös anzugehen und nicht nur rein symptomatisch wie mit den bisher verwendeten Antiphlogistika.

Darüber hinaus besteht die Möglichkeit, aufgrund der histologischen und immunologischen Parallelen zwischen allergischer Encephalomyelitis an Versuchstieren und der menschlichen multiplen Skelrose (vgl. hierzu: T.L. Willmon, Ann. N.Y. Acad. Sci. *122*, 1—2 (1965)) auch für diese bisher schwer therapeutisch angehbare Erkrankung eine spezifische Therapie mit dem erfindungsgemäßen Präparat einzuführen.

Arzneimittel gemäß der Erfindung enthalten den Wirkstoff der Formel I zur oralen Applikation, z.B. in Form von Kapseln, in Dosen von 25—150 mg, vorzugsweise 50—100 mg, zur rektalen Applikation, z.B. in Form von Suppositorien, in Dosen von 50—300 mg, vorzugsweise 100—200 mg. Diese Arzneimittel sind je nach Lage des Falles ein bis viermal, im Mittel zweimal täglich zu applizieren.

**Patentansprüche für die Vertragsstraten: BE, CH, DE, FR, GB, IT, LU, NL, SE.**

1. 5-Methylisoxazol-4-carbonsäure-(4-trifluormethyl)-anilid der Formel I

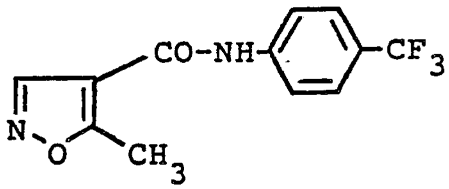

(I)

2. Verfahren zur Herstellung der Verbindung der Formel I, dadurch gekennzeichnet, daß man 4-Trifluormethylanilin der Formel II

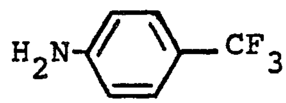

(II)

a) mit einem 5-Methylisoxazol-4-carbonsäure-Derivat der Formel III

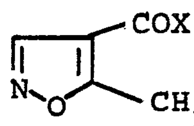

(III)

in der X entweder ein Halogenatom, vorzugsweise Chlor oder Brom, oder eine YO— oder ZO—CO—O—Gruppe bedeutet, wobei Y für gegebenenfalls durch Fluor, Chlor, Brom, Jod, Methyl, Äthyl, Methoxy, Äthoxy, Trifluormethyl, Nitro oder Cyan einfach, zweifach oder dreifach substituiertes Phenyl oder für den Acylrest entsprechend Formel III (Formel III) ohne X und Z für ($C_1$—$C_4$)-Alkyl, Phenyl oder Benzyl stehen, umsetzt, oder

b) mit Diketen oder einem reaktiven Acetessigsäure-derivat umsetzt, das so erhaltene Acetessigsäure-(4-trifluormethyl)-anilid der Formel IV

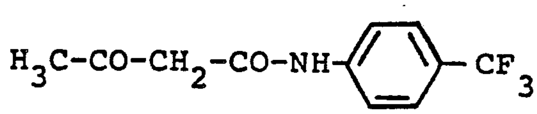

(IV)

mit einem Orthoameisensäureester der Formel V

$$HC(OR)_3 \qquad (V)$$

worin R ($C_1$—$C_4$)-Alkyl bedeutet, und mit einem Säureanhydrid erwärmt und das so erhaltene 2-Alkoxymethylenacetessigsäure-(4-trifluormethyl)-anilid der Formel VI

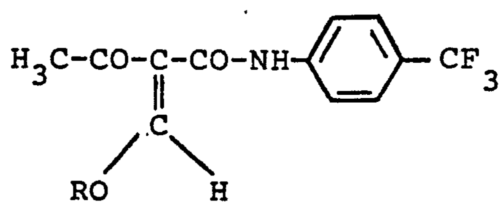

(VI)

worin R die oben gennanten Bedeutunge hat, mit Hydroxylamin umsetzt.

3. Arzneimittel, gekennzeichnet durch den Gehalt an der Verbindung nach Anspruch 1, in Mischung mit einem physiologisch verträglichen Trägerstoff und/oder Konstituens.

4. Verbindung nach Anspruch 1 zur Verwendung bei der Behandlung von Entzündungen, rheumatischen Beschwerden oder der multiplen Skelrose.

**Patentanspruch für den Verträgesstaat: AT**

Verfahren zur Herstellung von 5-Methylisoxazol-4-carbonsäure-(4-trifluormethyl)-anilid der Formel I

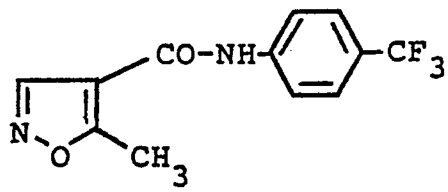

(I)

dadurch gekennzeichnet, daß man 4-Trifluormethylanilin der Formel II

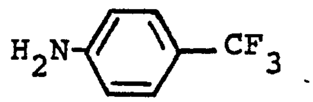

(II)

a) mit einem 5-Methylisoxazol-4-carbonsäure-Derivat der Formel III

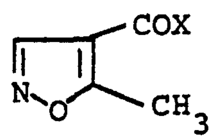

(III)

in der X entweder ein Halogenatom, vorzugsweise Chlor oder Brom, oder eine YO— oder ZO—CO—O—Gruppe bedeutet, wobei Y fur gegebenenfalls durch Fluor, Chlor, Brom, Jod, Methyl, Äthyl, Methoxy, Äthoxy, Trifluormethyl, Nitro oder Cyan einfach, zweifach oder dreifach substituiertes Phenyl oder für den Acylrest entsprechend Formel III (Formel III ohne X) und Z für ($C_1$—$C_4$)-Alkyl, Phenyl oder Benzyl stehen, umsetzt, oder

b) mit Diketen oder einem reaktiven Acetessigsäurederivat umsetzt, das so erhaltene Acetessigsäure-(4-trifluormethyl)-anilid der Formel IV

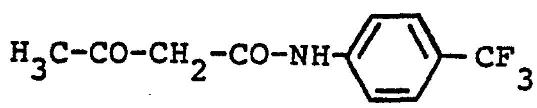

(IV)

mit einem Orthoameisensäureester der Formel V

$$HC(OR)_3 \qquad (V)$$

worin R ($C_1$—$C_4$)-Alkyl beudeutet, und mit einem Säure-anhydrid erwärmt und das so erhaltene 2-Alkoxymethylenacetessigsäure-(4-trifluormethyl)-anilid der Formel VI

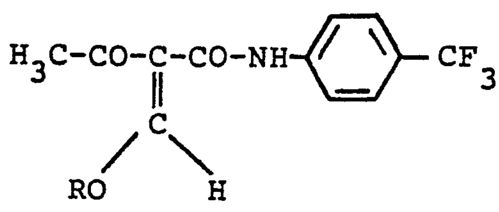

(VI)

worin R die oben genannten Bedeutungen hat, mit Hydroxylamin umsetzt.

**Claims for the Contracting States: BE, CH, DE, FR, GB, IT, LU, NL, SE.**

1. 5-Methylisoxazole-4-carboxylic acid-4-trifluoromethylanilide of the formula I

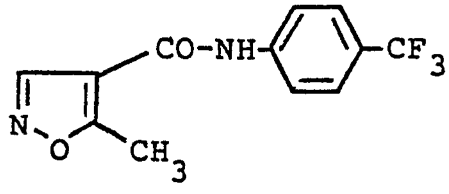

(I)

2. A process for the preparation of the compound of the formula I, wherein 4-trifluoromethylaniline of the formula II

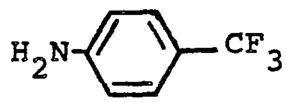

(II)

a) is reacted with a 5-methylisoxazole-4-carboxylic acid derivative of the formula III

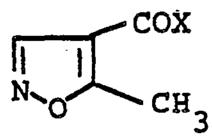

(III)

where X is either a halogen atom, preferably chlorine or bromine, or a YO— or ZO—CO—O— group, Y is phenyl which is optionally monosubstituted, disubstituted or trisubstituted by fluorine, chlorine, bromine, iodine, methyl, ethyl, methoxy, ethoxy, trifluoromethyl, nitro or cyano or is the acyl radical corresponding to formula III (namely formula III without X) and Z is ($C_1$—$C_4$)-alkyl, phenyl or benzyl, or b) is reacted with diketene or a reactive acetoacetic acid derivative, the resulting acetoacetic acid 4-trifluoromethyl-anilide of the formula IV

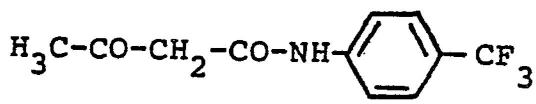

(IV)

is heated with an orthoformic acid ester of the formula V

$$HC(OR)^3 \quad (V)$$

wherein R is ($C_1$—$C_4$)-alkyl and with an acid anhydride, and the resulting 2-alkoxymethylene-acetoacetic acid 4-trifluoromethyl-anilide of the formula VI

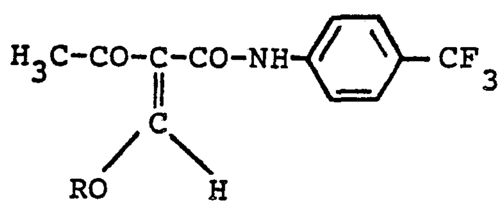

(VI)

wherein R has the above meanings, is reacted with hydroxyl amine.

3. A medicine containing the compound according to claim 1 in admixture with a physiologically compatible carrier and/or constituent.

4. Compound according to claim 1 for the treatment of inflammations, rheumatic complaints or multiple sclerosis.

**Claim for the Contracting State: AT**

A process for the preparation of 5-methylisoxazole-4-carboxylic acid-4-trifluoromethylanilide of the formula I

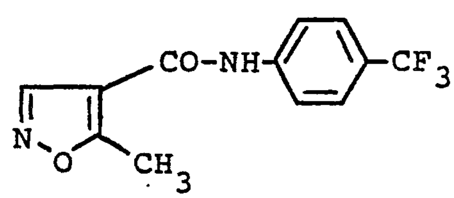

(I)

wherein 4-trifluoromethylanilide of the formula II

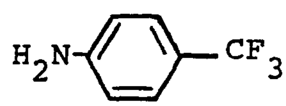

(II)

a) is reacted with a 5-Methylisoxazole-4-carboxylic acid derivative of the formula III

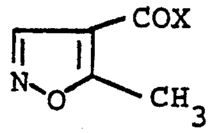

(III)

where X is either a halogen atom, preferably chlorine or bromine, or a YO— or ZO—CO—O— group, Y is phenyl which is optionally monosubstituted, disubstituted or trisubstituted by fluorine, chlorine, bromine, iodine, methyl, ethyl, methoxy, ethoxy, trifluoromethyl, nitro or cyano or is the acyl radical corresponding to formula III (namely formula III without X) and Z is ($C_1$—$C_4$)-alkyl, phenyl or benzyl, or

b) is reacted with diketene or a reactive acetoacetic acid derivative, the resulting acetoacetic acid 4-trifluoromethyl-anilide of the formula IV

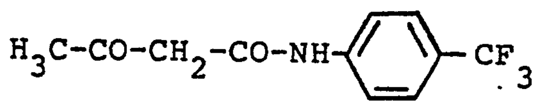

(IV)

is heated with an orthoformic acid ester of the formula V

$HC(OR)^3$ (V)

wherein R is ($C_1$—$C_4$)-alkyl and with an acid anhydride, and the resulting 2-alkoxymethylene-acetoacetic acid 4-trifluoromethyl-anilide of the formula VI

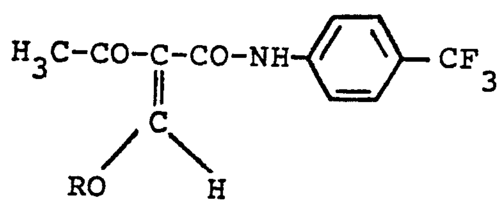

(VI)

wherein R has the above meanings, is reacted with hydroxyl amine.

**Revendications pour l'Etats contractants: BE, CH, DE, FR, GB, IT, LU, NL, SE.**

1. (4-trifluorométhyl)anilide de l'acide 5-méthylisoxazole-4-carboxylique de formule I:

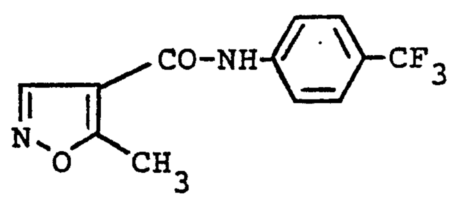

(I)

2. Procédé de préparation du composé de formule I, caractérisé en ce qu'on fait réagir la 4-trifluorométhyl-aniline de formule II:

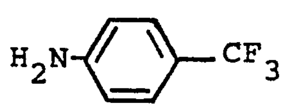

(II)

a) avec und dérivé de l'acide 5-méthyl-isoxazole-4-carboxylique de formule III:

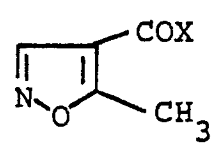

(III)

dans laquelle X représente un atome d'halogène, de préference le chlore ou le brome, ou un groupe YO— ou ZO—CO—O, auquel cas Y représente un groupe phényle ébentuellement substitué, une, deux

ou trois fois par le fluor, le chlore, le brome, l'iode, ou un groupe méthyle, éthyle, méthoxy, éthoxy, trifluorométhyle, nitro ou cyano, ou le radical acyle correspondant de formule III (formule III sans X) et Z représente un groupe alkyle en $C_1$—$C_4$, phényle ou benzyle, ou

b) avec un dicétène ou un dérivé réactif de l'acide acétlyacétique, on chauffe le (4-trifluorométhyl)anilide de l'acide acétylacétique de formule IV ainsi obtenu:

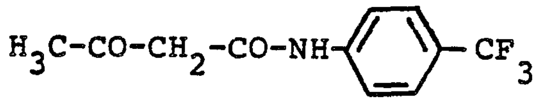 (IV)

avec un ester de l'acide orthoformique de formule V:

$$HC(OR)_3 \quad (V)$$

où R représente un groupe alkyle en $C_1$—$C_4$, et avec un anhydride d'acide et on fait réagir le (4-trifluorométhyl)anilide de l'acide 2-alcoxy-méthylèneacétylacétique de formule VI ainsi obtenu:

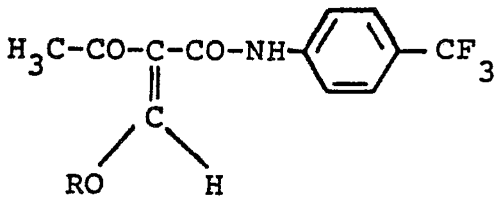 (VI)

où R a la signification indiquée ci-dessus, avec l'hydroxylamine.

3. Médicament, caractérisé en ce qu'il contient le composé selon la revendication 1, associé avec un véhicule et/ou support physiologiquement compatibles.

4. Composé selon la revendication 1 pour utilisation dans le traitement de maladies inflammatoires, des atteintes rhumatismales ou de la sclérose en plaques.

**Revendication pour l'Etat contractant: AT**

Procédé de préparation du (4-trifluorométhyl)anilide de l'acide 5-méthylisoxazole-4-carboxylique de formule I

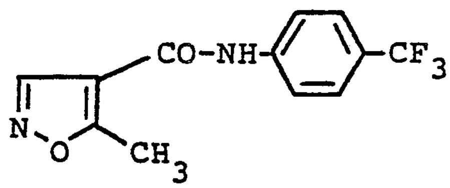 (I)

caractérisé en ce qu'on fait réagir la 4-trifluorométhylaniline de formule II

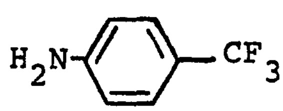 (II)

a) avec un dérivé de l'acide 5-méthylisoxazole-4-carboxylique de formule III

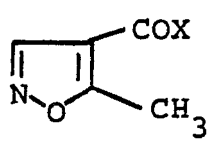 (III)

dans laquelle X représente un atome d'halogène, de préférence le chlore ou le brome, ou un groupe YO— ou ZO—CO—O, auquel cas Y représente un groupe phényle éventuellement substitué une, deux ou trois fois par le fluor, le chlore, le brome, l'iode ou un groupe méthyle, éthyle, méthoxy, éthoxy, trifluorométhyle, nitro ou cyano, ou le radical acyle correspondant à la formule III (formule III sans X) et Z représente un groupe alkyle en $C_1$—$C_4$, phényle ou benzyle, ou

b) avec un dicétène ou un dérivé réactif de l'acide acétylacétique, on chauffe le (4-trifluorométhyl)anilide de l'acide acétylacétique de formule IV, ainsi obtenu

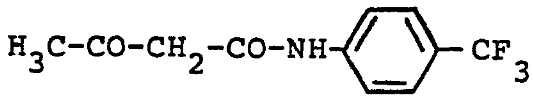

(IV)

avec un ester de l'acide orthoformique de formule V

$HC(OR)_3$ (V)

où R représente un groupe alkyle en $C_1$—$C_4$, et avec un anhydride d'acide, et on fait réagir le (4-trifluorométhyl)-anilide de l'acide 2-alcoxyméthlèneacétylacétique de formule VI, ainsi obtenu

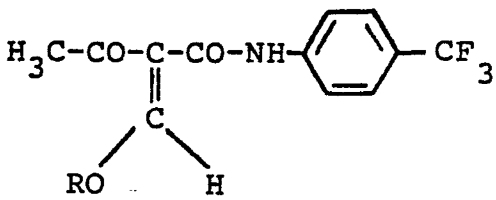

(VI)

où R a la signification indiquée ci-dessus, avec l'hydroxylamine.